# EUROPEAN PATENT APPLICATION

(11) **EP 4 813 963 A1**
(43) Date of publication of application: **30.09.2026**
(21) Application number: 26730388.1
(22) Date of filing: 28.01.2026
(51) Int. Cl.: C07C 17/358, C07C 17/38, C07C 21/22, C09K 5/04, F25B 1/00

(54) **METHOD FOR PRODUCING HEXAFLUORO-2-BUTYNE**

(30) Priority: 31.01.2025 JP 2025015486; 31.01.2025 JP 2025015489; 31.01.2025 JP 2025015493
(71) Applicant: DAIKIN INDUSTRIES, LTD., Osaka-shi, Osaka 530-0001 (JP)
(72) Inventor: ETO, Yusuke, Osaka-shi, Osaka 530-0001 (JP); NAKAMURA, Shingo, Osaka-shi, Osaka 530-0001 (JP); KUROKI, Yoshichika, Osaka-shi, Osaka 530-0001 (JP); SUGIYAMA, Akinari, Osaka-shi, Osaka 530-0001 (JP)
(74) Representative: Hoffmann Eitle
(86) International application number: PCT/JP2026/002930
(87) International publication number: WO 2026/164177

(57) **Abstract**

An object of the present disclosure is to newly provide a method for producing hexafluoro-2-butyne. Provided is a method for producing hexafluoro-2-butyne.

## Description

### Technical Field

The present disclosure relates to a method for producing hexafluoro-2-butyne.

### Background Art

PTL 1 discloses a method for producing a perfluoroalkyne compound, comprising reacting a perfluoroalkadiene compound in the presence of a catalyst to obtain a perfluoroalkyne compound; a composition comprising a perfluoroalkyne compound and a perfluorocycloalkene compound; a catalyst for use in reacting a perfluoroalkadiene compound to obtain a perfluoroalkyne compound; and a method for producing a catalyst for use in reacting a perfluoroalkadiene compound to obtain a perfluoroalkyne compound.

PTL 2 discloses a method for producing a halogenated butene compound, comprising subjecting a halogenated butane compound to a dehydrofluorination reaction; a method for producing a halogenated butyne compound, comprising subjecting a halogenated butene compound to a dehydrohalogenation reaction; and a method for producing a halogenated butyne compound, comprising subjecting a halogenated butane compound to a dehydrofluorination reaction to produce a halogenated butene compound and then subjecting the halogenated butene compound to a dehydrohalogenation reaction to produce a halogenated butyne compound.

PTL 3 discloses a method for producing a perfluorocarbon compound, comprising reacting a halogenated hydrocarbon compound in an organic solvent in the presence of an iodine-containing inorganic material and zinc or a zinc alloy to obtain a perfluorocarbon compound; and a method for producing a perfluoroalkyne compound, comprising reacting a halogenated alkene compound in at least one nitrogen-containing polar organic solvent selected from the group consisting of N,N-dimethylacetamide, N,N-diisopropylformamide, N-methyl-2-pyrrolidone, 1,3-dimethyl-3,4,5,6-tetrahydropyrimidinone, hexamethylphosphoric triamide, amine compounds, pyridine compounds, and quinoline compounds in the presence of zinc or a zinc alloy to obtain a perfluoroalkyne compound.

### Citation List

### Patent Literature

PTL 1: WO2020/075728A1
PTL 2: WO2020/171011A1
PTL 3: WO2020/204146A1

### Summary of Invention

### Technical Problem

An object of the present disclosure is to newly provide a method for producing hexafluoro-2-butyne.

### Solution to Problem

The present disclosure includes the following subject matter.

### Item 1.

A method for producing (1) 1,1,1,4,4,4-hexafluoro-2-butyne (CF₃C≡CCF₃), comprising
subjecting (2) 1,1,2,3,4,4-hexafluoro-1,3-butadiene (CF₂=CF-CF=CF₂) to an isomerization reaction
(3) in the presence of
(3-1) at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br),
(3-2) oxygen, or
(3-3) water.

### Item 2.

The production method according to Item 1, wherein in the isomerization reaction step,
when the isomerization reaction step is performed (3) in the presence of (3-1) the olefin compound, the content of the 1,1,2,3,4,4-hexafluoro-1,3-butadiene (CF₂=CF-CF=CF₂) is 99.0 vol% or more and 99.999 vol% or less, and the content of the olefin compound is 0.001 vol% or more and 1 vol% or less, based on the total amount of a composition comprising the 1,1,2,3,4,4-hexafluoro-1,3-butadiene (CF₂=CF-CF=CF₂) and the olefin compound;
when the isomerization reaction step is performed (3) in the presence of (3-2) the oxygen, the content of the 1,1,2,3,4,4-hexafluoro-1,3-butadiene (CF₂=CF-CF=CF₂) is 95 vol% or more and 99.999 vol% or less, and the content of the oxygen is 0.01 vol ppm or more and 10 vol ppm or less, based on the total amount of a composition comprising the 1,1,2,3,4,4-hexafluoro-1,3-butadiene (CF₂=CF-CF=CF₂) and the oxygen; or
when the isomerization reaction step is performed (3) in the presence of (3-3) the water, the content of the 1,1,2,3,4,4-hexafluoro-1,3-butadiene (CF₂=CF-CF=CF₂) is 95 vol% or more and 99.999 vol% or less, and the content of the water is 0.01 vol ppm or more and 50 vol ppm or less, based on the total amount of a composition comprising the 1,1,2,3,4,4-hexafluoro-1,3-butadiene (CF₂=CF-CF=CF₂) and the water.

### Item 3.

The production method according to Item 1, wherein the isomerization reaction step is performed in a liquid phase.

### Item 4.

A composition comprising:
(1) 1,1,1,4,4,4-hexafluoro-2-butyne (CF₃C≡CCF₃);
(2) 1,1,2,3,4,4-hexafluoro-1,3-butadiene (CF₂=CF-CF=CF₂); and
(3) at least one component selected from the group consisting of
   (3-1) at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br),
   (3-2) oxygen, and
   (3-3) water.

### Item 5.

The composition according to Item 4, wherein based on the total amount of the composition,
the content of (1) the 1,1,1,4,4,4-hexafluoro-2-butyne (CF₃C≡CCF₃) is 95 vol% or more and 99.99999 vol% or less,
the content of (2) the 1,1,2,3,4,4-hexafluoro-1,3-butadiene (CF₂=CF-CF=CF₂) is 0.01 vol% or more and 1 vol% or less, and
as (3) the at least one component,
when the composition comprises (3-1) the olefin compound, the content of the olefin compound is 0.01 vol% or more and 1.5 vol% or less,
when the composition comprises (3-2) the oxygen, the content of the oxygen is 0.01 vol ppm or more and 10 vol ppm or less; or
when the composition comprises (3-3) the water, the content of the water is 0.01 vol ppm or more and 30 vol ppm or less.

### Item 6.

The composition according to Item 4 for use as an etching gas, a refrigerant, a heat-transfer medium, a deposit gas, a building block for organic synthesis, or a cleaning gas.

The present disclosure discloses a method for producing hexafluoro-2-butyne (CF₃C≡CCF₃). According to the present disclosure, hexafluoro-2-butyne is produced by subjecting hexafluoro-1,3-butadiene (CF₂=CF-CF=CF₂) to an isomerization reaction in the presence of an olefin compound, such as heptafluorobutene (C₄F₇H), in the presence of oxygen, or in the presence of water, whereby polymerization (formation of polymers) or decomposition (formation of decomposed products) of hexafluoro-2-butyne is suppressed, or formation of acid components is suppressed during storage or transportation of the produced composition comprising hexafluoro-2-butyne in cylinders or the like, thereby a decrease in purity of hexafluoro-2-butyne is suppressed while maintaining the storage stability of the composition comprising hexafluoro-2-butyne.

The composition comprising hexafluoro-2-butyne of the present disclosure contains an olefin compound, such as heptafluorobutene (C₄F₇H), oxygen, or water, and has improved storage stability. Thus, even under harsh maritime transportation conditions in containers or the like (e.g., temperature rise during transportation), polymerization (formation of polymers) or decomposition (formation of decomposed products) of hexafluoro-2-butyne is suppressed, or formation of acid components is suppressed, whereby the composition comprising hexafluoro-2-butyne can be stably stored and transported.

### Advantageous Effects of Invention

The present disclosure can newly provide a method for producing hexafluoro-2-butyne.

### Description of Embodiments

In the present specification, the terms "comprise," "contain," and "include" encompass the concepts of comprising, consisting essentially of, and consisting of. In the present specification, a numerical range indicated by "A to B" means A or more and B or less. In the present specification, expressions such as "part(s)" and "%" denote part(s) by mass, part(s) by weight, mass percent (mass%), or weight percent (wt%).

### [1] Method for Producing Hexafluoro-2-butyne

In the method for producing (1) 1,1,1,4,4,4-hexafluoro-2-butyne (CF₃C≡CCF₃) of the present disclosure, (2) 1,1,2,3,4,4-hexafluoro-1,3-butadiene (CF₂=CF-CF=CF₂) is included in the starting material, and the concentration of (3) (3-1) an olefin compound, (3-2) oxygen (O₂), or (3-3) water is adjusted, whereby polymerization (e.g., dimerization) of the halogenated alkyne compound or formation of acid components can be suppressed in the production of 1,1,1,4,4,4-hexafluoro-2-butyne, thereby improving the storage stability of the crude product after the reaction.

In the method for producing 1,1,1,4,4,4-hexafluoro-2-butyne (CF₃C≡CCF₃) of the present disclosure, the concentration of the olefin compound, oxygen (O₂), or water in the crude 1,1,1,4,4,4-hexafluoro-2-butyne obtained after the reaction is adjusted, whereby decomposition reactions, as well as formation of polymers and acid components, can be suppressed during storage in a storage tank, thereby suppressing, for example, clogging of pipes and corrosion of equipment during storage in the storage tank.

The method for producing 1,1,1,4,4,4-hexafluoro-2-butyne (CF₃C≡CCF₃) of the present disclosure comprises:
subjecting 1,1,2,3,4,4-hexafluoro-1,3-butadiene (CF₂=CF-CF=CF₂) to an isomerization reaction in the presence of at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br).

In the isomerization reaction step of the method for producing 1,1,1,4,4,4-hexafluoro-2-butyne (CF₃C≡CCF₃) of the present disclosure, it is preferred that
the content of the 1,1,2,3,4,4-hexafluoro-1,3-butadiene (CF₂=CF-CF=CF₂) is 99.0 vol% or more and 99.999 vol% or less, and the content of the olefin compound is 0.001 vol% or more and 1 vol% or less, based on the total amount of a composition comprising the 1,1,2,3,4,4-hexafluoro-1,3-butadiene (CF₂=CF-CF=CF₂) and the olefin compound.

The method for producing 1,1,1,4,4,4-hexafluoro-2-butyne (CF₃C≡CCF₃) of the present disclosure comprises:
subjecting 1,1,2,3,4,4-hexafluoro-1,3-butadiene (CF₂=CF-CF=CF₂) to an isomerization reaction in the presence of oxygen.

In the isomerization reaction step of the method for producing 1,1,1,4,4,4-hexafluoro-2-butyne (CF₃C≡CCF₃) of the present disclosure, it is preferred that
the content of the 1,1,2,3,4,4-hexafluoro-1,3-butadiene (CF₂=CF-CF=CF₂) is 95 vol% or more and 99.999 vol% or less, and the content of the oxygen is 0.01 vol ppm or more and 10 vol ppm or less, based on the total amount of a composition comprising the 1,1,2,3,4,4-hexafluoro-1,3-butadiene (CF₂=CF-CF=CF₂) and the oxygen.

The method for producing 1,1,1,4,4,4-hexafluoro-2-butyne (CF₃C≡CCF₃) of the present disclosure comprises:
subjecting 1,1,2,3,4,4-hexafluoro-1,3-butadiene (CF₂=CF-CF=CF₂) to an isomerization reaction in the presence of water.

In the isomerization reaction step of the method for producing 1,1,1,4,4,4-hexafluoro-2-butyne (CF₃C≡CCF₃) of the present disclosure, it is preferable that
the content of the 1,1,2,3,4,4-hexafluoro-1,3-butadiene (CF₂=CF-CF=CF₂) is 95 vol% or more and 99.999 vol% or less, and the content of the water is 0.01 vol ppm or more and 50 vol ppm or less, based on the total amount of a composition comprising the 1,1,2,3,4,4-hexafluoro-1,3-butadiene (CF₂=CF-CF=CF₂) and the water.

In the method for producing 1,1,1,4,4,4-hexafluoro-2-butyne (CF₃C≡CCF₃) of the present disclosure, the isomerization reaction step is preferably performed in a liquid phase.

In the production of hexafluoro-2-butyne of the present disclosure, hexafluoro-1,3-butadiene (CF₂=CF-CF=CF₂) is subjected to an isomerization reaction in the presence of (3-1) an olefin compound, such as heptafluorobutene (C₄F₇H), (3-2) oxygen (O₂), or (3-3) water, whereby polymerization (formation of polymers) or decomposition (formation of decomposed products) of hexafluoro-2-butyne is suppressed, or formation of acid components is suppressed during storage or transportation of the produced composition comprising hexafluoro-2-butyne in cylinders or the like, thereby a decrease in purity of hexafluoro-2-butyne is suppressed while maintaining the storage stability of the composition comprising hexafluoro-2-butyne.

In the present disclosure, the term "conversion rate" means the ratio (mol%) of the total molar amount of compounds other than the starting compound contained in the outflow gas from a reactor outlet (e.g., 1,1,1,4,4,4-hexafluoro-2-butyne, which is the target compound) to the molar amount of the starting compound (1,1,2,3,4,4-hexafluoro-1,3-butadiene) supplied to the reactor.

In the present disclosure, the term "selectivity" means the ratio (mol%) of the total molar amount of the target compound (1,1,1,4,4,4-hexafluoro-2-butyne) contained in the outflow gas from a reactor outlet to the total molar amount of compounds other than the starting compound in the outflow gas from the reactor outlet (e.g., 1,1,1,4,4,4-hexafluoro-2-butyne, which is the target compound).

### (1) Starting Compound

In the present disclosure, the starting compound is 1,1,2,3,4,4-hexafluoro-1,3-butadiene (CF₂=CF-CF=CF₂).

### (2) Isomerization Reaction

### (2-1) In the Presence of Olefin Compound

In the isomerization reaction step of the present disclosure, 1,1,2,3,4,4-hexafluoro-1,3-butadiene (CF₂=CF-CF=CF₂) is subjected to an isomerization reaction in the presence of at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br)to thereby produce 1,1,1,4,4,4-hexafluoro-2-butyne (CF₃C≡CCF₃).

The isomerization reaction step is preferably performed in a liquid phase.

### Olefin Compound

The olefin compound is at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br).

### Octafluorobutene

For octafluorobutene (C₄F₈), any isomer may be used. The octafluorobutene is preferably octafluoro-1-butene, (E/Z)-octafluoro-2-butene, (E)-octafluorobutene ((E)-octafluoro-2-butene), (Z)-octafluorobutene ((Z)-octafluoro-2-butene), octafluorocyclobutane, pentafluorotrifluoromethylpropene, or the like.

The octafluorobutene may contain isomers, and may be used singly or as a mixture (blend) of two or more types.

### Heptafluorobutene

For heptafluorobutene (C₄F₇H), any isomer may be used. The heptafluorobutene is preferably 1,1,2,3,3,4,4-heptafluoro-1-butene, (E/Z)-1,1,1,2,4,4,4-heptafluoro-2-butene, (E)-1,1,1,2,4,4,4-pentafluoro-2-butene, (Z)-1,1,1,2,4,4,4-pentafluoro-2-butene, 1,1,2,3,3,4,4-heptafluoro-1-butene, 1,2,3,3,4,4,4-heptafluoro-1-butene, 1,1,3,3,4,4,4-heptafluoro-1-butene, 1,1,2,3,4,4,4-heptafluoro-1-butene, 1,3,3,3-tetrafluoro-2-(trifluoromethyl)-1-propene, 1,1,1,2,3,4,4-heptafluoro-2-butene, or the like.

The heptafluorobutene may contain isomers, and may be used singly or as a mixture (blend) of two or more types.

### Pentafluorobutadiene

For pentafluorobutadiene (C₄F₅H), any isomer may be used. The pentafluorobutadiene is preferably 1,1,2,4,4-pentafluoro-1,3-butadiene, 1,3,3,4,4-pentafluorooctylcyclobutene, 3,3,4,4,4-pentafluoro-1-butyne, 1,2,3,3,4-pentafluorocyclobutene, 1,1,2,3,4-pentafluoro-1,3-butadiene, 3,3-difluoro-1-(trifluoromethyl)cyclopropene, 1,2,2,4,4-pentafluorobicyclo[1.1.0]butane, 1,1,1,4,4-pentafluoro-2-butyne, or the like.

The pentafluorobutadiene may contain isomers, and may be used singly or as a mixture (blend) of two or more types.

### Chloropentafluorobutadiene

For chloropentafluorobutadiene (C₄F₅Cl), any isomer may be used. The chloropentafluorobutadiene is preferably (E/Z)-1-chloropentafluoro-1,3-butadiene, (E)-1-chloropentafluoro-1,3-butadiene, (Z)-1-chloropentafluoro-1,3-butadiene, 1-chloro-2,3,3,4,4-pentafluorocyclobutene, 2-chloropentafluoro-1,3-butadiene, 3-chloro-1,2,3,4,4-pentafluorocyclobutene, 1-chloro-1,2,3,4,4-pentafluoro-1,3-butadiene, 1-chloro-3,3,4,4,4-pentafluoro-1-butyne, or the like.

The chloropentafluorobutadiene may contain isomers, and may be used singly or as a mixture (blend) of two or more types.

### Bromotrifluoroethylene

For bromotrifluoroethylene (C₂F₃Br), 1-bromo-1,2,2-trifluoroethene may be used.

### Preferred Olefin Compound

The olefin compound is preferably at least one olefin compound selected from the group consisting of
octafluorobutene, such as octafluoro-1-butene and (E/Z)-octafluoro-2-butene;
heptafluorobutene, such as 1,1,2,3,3,4,4-heptafluoro-1-butene and (E/Z)-1,1,1,2,4,4,4-heptafluoro-2-butene;
pentafluorobutadiene, such as 2H-pentafluoro-1,3-butadiene;
chloropentafluorobutadiene, such as (E/Z)-1-chloropentafluoro-1,3-butadiene; and
bromotrifluoroethylene, such as 1-bromo-1,2,2-trifluoroethene; and more preferably
heptafluorobutene (C₄F₇H), such as 1,1,2,3,3,4,4-heptafluoro-1-butene or (E/Z)-1,1,1,2,4,4,4-heptafluoro-2-butene.

### Composition of the Composition During Isomerization Reaction 1,1,2,3,4,4-Hexafluoro-1,3-butadiene

In the isomerization reaction step of the method for producing 1,1,1,4,4,4-hexafluoro-2-butyne (CF₃C≡CCF₃) of the present disclosure, the content of 1,1,2,3,4,4-hexafluoro-1,3-butadiene is preferably adjusted to 99 vol% or more and 99.999 vol% or less, more preferably 99.5 vol% or more and 99.995 vol% or less, even more preferably 99.9 vol% or more and 99.992 vol% or less, and particularly preferably 99.95 vol% or more and 99.99 vol% or less, based on the total amount of the composition comprising 1,1,2,3,4,4-hexafluoro-1,3-butadiene (CF₂=CF-CF=CF₂) and the olefin compound.

### Olefin Compound

In the isomerization reaction step of the method for producing 1,1,1,4,4,4-hexafluoro-2-butyne (CF₃C≡CCF₃) of the present disclosure, the content of the olefin compound is preferably adjusted to 0.001% by volume (vol%) or more and 1% by volume (vol%) or less, more preferably adjusted to 0.005 vol% or more and 0.75 vol% or less, even more preferably adjusted to 0.01 vol% or more and 0.5 vol% or less, and particularly preferably adjusted to 0.05 vol% or more and 0.35 vol% or less, based on the total amount of the composition comprising 1,1,2,3,4,4-hexafluoro-1,3-butadiene (CF₂=CF-CF=CF₂) and the olefin compound.

In the method for producing 1,1,1,4,4,4-hexafluoro-2-butyne (CF₃C≡CCF₃) of the present disclosure, the concentration of the olefin compound included in the starting material is adjusted, whereby polymerization (e.g., dimerization) of the halogenated alkyne compound or formation of acid components can be suppressed, thereby improving the storage stability of the crude product after the reaction.

In the method for producing 1,1,1,4,4,4-hexafluoro-2-butyne (CF₃C≡CCF₃) of the present disclosure, the concentration of the olefin compound in the crude 1,1,1,4,4,4-hexafluoro-2-butyne obtained after the reaction is adjusted, whereby decomposition reactions, as well as formation of polymers and acid components, can be suppressed during storage in a storage tank, thereby suppressing, for example, clogging of pipes and corrosion of equipment during storage in the storage tank.

### (2-2) In the Presence of Oxygen

In the isomerization reaction step of the present disclosure, 1,1,2,3,4,4-hexafluoro-1,3-butadiene (CF₂=CF-CF=CF₂) is subjected to an isomerization reaction in the presence of oxygen to thereby produce 1,1,1,4,4,4-hexafluoro-2-butyne (CF₃C=CCF₃).

The isomerization reaction step is preferably performed in a liquid phase.

### Composition of the Composition During Isomerization Reaction 1,1,2,3,4,4-Hexafluoro-1,3-butadiene

In the isomerization reaction step of the method for producing 1,1,1,4,4,4-hexafluoro-2-butyne (CF₃C≡CCF₃) of the present disclosure, the content of 1,1,2,3,4,4-hexafluoro-1,3-butadiene is preferably adjusted to 95 vol% or more and 99.999 vol% or less, more preferably adjusted to 96 vol% or more and 99.995 vol% or less, even more preferably adjusted to 97 vol% or more and 99.992 vol% or less, and particularly preferably adjusted to 98 vol% or more and 99.99 vol% or less, based on the total amount of the composition comprising 1,1,2,3,4,4-hexafluoro-1,3-butadiene (CF₂=CF-CF=CF₂) and oxygen.

### Oxygen

In the isomerization reaction step of the method for producing 1,1,1,4,4,4-hexafluoro-2-butyne (CF₃C≡CCF₃) of the present disclosure, the content of oxygen is preferably adjusted to 0.01 ppm by volume (vol ppm) or more and 10 ppm by volume (vol ppm) or less, more preferably 0.1 vol ppm or more and 9.5 vol ppm or less, even more preferably 0.5 vol ppm or more and 7.5 vol ppm or less, and particularly preferably 1 vol ppm or more and 5 vol ppm or less, based on the total amount of the composition comprising 1,1,2,3,4,4-hexafluoro-1,3-butadiene (CF₂=CF-CF=CF₂) and oxygen.

In the method for producing 1,1,1,4,4,4-hexafluoro-2-butyne (CF₃C≡CCF₃) of the present disclosure, the concentration of oxygen included in the starting material is adjusted, whereby polymerization (e.g., dimerization) of the halogenated alkyne compound or formation of acid components can be suppressed, thereby improving the storage stability of the crude product after the reaction.

In the method for producing 1,1,1,4,4,4-hexafluoro-2-butyne (CF₃C≡CCF₃) of the present disclosure, the concentration of oxygen in the crude 1,1,1,4,4,4-hexafluoro-2-butyne obtained after the reaction is adjusted, whereby decomposition reactions, as well as formation of polymers and acid components, can be suppressed during storage in a storage tank, thereby suppressing, for example, clogging of pipes and corrosion of equipment during storage in the storage tank.

### (2-3) In the presence of Water

In the isomerization reaction step of the present disclosure, 1,1,2,3,4,4-hexafluoro-1,3-butadiene (CF₂=CF-CF=CF₂) is subjected to an isomerization reaction in the presence of water to thereby produce 1,1,1,4,4,4-hexafluoro-2-butyne (CF₃C=CCF₃).

The isomerization reaction step is preferably performed in a liquid phase.

### Composition of the Composition During Isomerization Reaction 1,1,2,3,4,4-Hexafluoro-1,3-butadiene

In the isomerization reaction step of the method for producing 1,1,1,4,4,4-hexafluoro-2-butyne (CF₃C≡CCF₃) of the present disclosure, the content of 1,1,2,3,4,4-hexafluoro-1,3-butadiene is preferably adjusted to 95 vol% or more and 99.999 vol% or less, more preferably 96 vol% or more and 99.995 vol% or less, even more preferably 97 vol% or more and 99.992 vol% or less, and particularly preferably 98 vol% or more and 99.99 vol% or less, based on the total amount of the composition comprising 1,1,2,3,4,4-hexafluoro-1,3-butadiene (CF₂=CF-CF=CF₂) and water.

### Water

In the isomerization reaction step of the method for producing 1,1,1,4,4,4-hexafluoro-2-butyne (CF₃C≡CCF₃) of the present disclosure, the content of water is preferably adjusted to 0.01 ppm by volume (vol ppm) or more and 50 ppm by volume (vol ppm) or less, more preferably adjusted to 0.05 vol ppm or more and 40 vol ppm or less, even more preferably adjusted to 0.5 vol ppm or more and 30 vol ppm or less, and particularly preferably adjusted to 1 vol ppm or more and 20 vol ppm or less, based on the total amount of the composition comprising 1,1,2,3,4,4-hexafluoro-1,3-butadiene (CF₂=CF-CF=CF₂) and water.

In the method for producing 1,1,1,4,4,4-hexafluoro-2-butyne (CF₃C≡CCF₃) of the present disclosure, the concentration of water included in the starting material is adjusted, whereby polymerization (e.g., dimerization) of the halogenated alkyne compound or formation of acid components can be suppressed, thereby improving the storage stability of the crude product after the reaction.

In the method for producing 1,1,1,4,4,4-hexafluoro-2-butyne (CF₃C≡CCF₃) of the present disclosure, the concentration of water in the crude 1,1,1,4,4,4-hexafluoro-2-butyne obtained after the reaction is adjusted, whereby decomposition reactions, as well as formation of polymers and acid components, can be suppressed during storage in a storage tank, thereby suppressing, for example, clogging of pipes and corrosion of equipment during storage in the storage tank.

### Lewis Acid Catalyst (Fluorination Catalyst or Non-fluorination Catalyst)

In the isomerization reaction step of the present disclosure, preferably, a Lewis acid catalyst may be used as a catalyst.

In the isomerization reaction step, the catalyst is preferably a fluorinated Lewis acid catalyst or a non-fluorinated Lewis acid catalyst.

The Lewis acid catalyst is preferably an aluminum chloride catalyst, a chromium oxide catalyst, an alumina catalyst, a silica-alumina catalyst, a zeolite catalyst, or the like. The Lewis acid catalyst may be a non-fluorinated Lewis acid catalyst or a fluorinated Lewis acid catalyst.

### Aluminum Chloride Catalyst

The aluminum chloride catalyst may be fluorinated. The fluorinated aluminum chloride can be obtained by reacting aluminum chloride with a fluorinating agent.

### Chromium Oxide Catalyst

The chromium oxide catalyst is preferably a catalyst represented by CrOₘ wherein 1.5 < m < 3, more preferably 2 < m < 2.75, and even more preferably 2 < m < 2.3. When chromium oxide is represented by CrOₘ·nH₂0, n is preferably 3 or less, and more preferably 1 to 1.5.

The fluorinated chromium oxide catalyst is preferably prepared by fluorination of a chromium oxide catalyst. The fluorination is preferably performed using, for example, HF or fluorocarbon. The fluorinated chromium oxide catalyst is synthesized, for example, by the method described in JPH05-146680A.

### Alumina Catalyst

The alumina catalyst is preferably α-alumina, activated alumina, or the like. The activated alumina is preferably ρ-alumina, χ-alumina, κ-alumina, η-alumina, pseudo-γ-alumina, γ-alumina, σ-alumina, θ-alumina, or the like. The alumina catalyst can also be a composite oxide containing alumina and another compound.

The composite oxide is preferably a silica-alumina catalyst. The silica-alumina catalyst is a composite oxide catalyst containing silica (SiO₂) and alumina (Al₂O₃). The catalyst to be used preferably has a silica content of 20 to 90 mass%, more preferably 50 to 80 mass%, based on the total amount of silica and alumina taken as 100 mass%.

Alumina catalysts and silica-alumina catalysts show higher activity when fluorinated. Preferably, alumina catalysts are fluorinated in advance before use for a catalytic reaction and are used as a fluorinated alumina catalyst. Silica-alumina catalysts are preferably fluorinated in advance before use for a catalytic reaction and are used as a fluorinated silica-alumina catalyst.

The fluorinating agent that is used to fluorinate an alumina catalyst or a silica-alumina catalyst is preferably, for example, an inorganic fluorinating agent, such as F₂ or HF, or a fluorocarbon-based organic fluorinating agent, such as hexafluoropropene.

The method for fluorinating an alumina catalyst and a silica-alumina catalyst is preferably a fluorination method comprising circulating a fluorinating agent through the catalyst under pressure conditions in the range of -50 kPa to 2.0 MPa and temperature conditions in the range of about -20°C to 400°C.

The method for fluorinating an alumina catalyst and a silica-alumina catalyst is preferably a fluorination method comprising circulating a fluorinating agent through the catalyst at atmospheric pressure under temperature conditions in the range of room temperature (25°C) to about 400°C, or a fluorination method comprising confining a fluorinating agent under pressure conditions in the range of -50 kPa to 2,000 kPa and temperature conditions in the range of about -20°C to 400°C.

### Zeolite Catalyst

The zeolite catalyst can be selected from a wide range of known types of zeolite. The zeolite catalyst is preferably a crystalline hydrous aluminosilicate of an alkali metal or an alkaline earth metal. The crystalline form of zeolite is preferably type A, type X, type LSX, or the like. The alkali metal or alkali earth metal in zeolite is preferably, for example, potassium, sodium, calcium, or lithium.

Zeolite catalysts show higher activity when fluorinated. The zeolite catalysts are preferably fluorinated in advance before use for a catalytic reaction and are used as a fluorinated zeolite catalyst.

The fluorinating agent used to fluorinate a zeolite catalyst is preferably, for example, an inorganic fluorinating agent, such as F₂ or HF, or a fluorocarbon-based organic fluorinating agent, such as hexafluoropropene.

The method for fluorinating the zeolite catalyst is preferably a fluorination method comprising circulating a fluorinating agent through the catalyst under pressure conditions in the range of -50 kPa to 2.0 MPa and temperature conditions in the range of about -20°C to 400°C.

The method for fluorinating the zeolite catalyst is preferably a fluorination method comprising circulating a fluorinating agent through the catalyst at atmospheric pressure under temperature conditions in the range of room temperature (25°C) to about 400°C.

### Titanium Oxide Catalyst

The titanium oxide catalyst preferably contains titanium dioxide as a main component and may further contain one or more non-volatile substances, such as other metal oxides, hydroxides, sulfates, nitrates, phosphates, and sulfides. The titanium dioxide content of the titanium oxide catalyst is preferably 70 mass% or more.

The titanium dioxide is preferably anatase titanium dioxide. The specific surface area is preferably 5 m²/g to 100 m²/g, and the pore volume is preferably 0.2 mL/g to 0.4 mL/g. The catalyst is preferably formed into a spherical shape. For example, commercially available products, such as CS-200, CS-300, and CS-950, can be used.

The catalyst is preferably a fluorinated titanium oxide catalyst because it exhibits high activity for isomerization reactions, increases the conversion rate of the reaction, and easily reduces catalyst degradation, and also because catalyst degradation can be easily inhibited even when an isomerization reaction is performed over a long period of time.

### Iron Oxide Catalyst

Iron oxide catalysts that can be used include iron catalysts, such as iron oxide, fluorinated iron oxide, and iron fluoride.

The catalysts may be used singly or as a mixture (blend) of two or more types.

### Fluorination of Catalyst

In the isomerization reaction step of the present disclosure, a fluorinated metal oxide catalyst is preferably used as the catalyst. When fluorinated, the catalyst shows higher activity and catalyst degradation tends to be easily reduced. By adjusting the pore volume of the catalyst, catalyst degradation can be inhibited even when the isomerization reaction is performed over a long period of time.

The method for fluorinating a metal oxide catalyst preferably comprises reacting a metal oxide with a fluorinating agent. Specifically, a metal oxide is fluorinated by circulating a fluorinating agent through the metal oxide. The metal oxide used in this fluorination is a metal oxide of the fluorinated metal oxide described above.

Preferable examples of fluorinating agents include hydrofluorocarbons (R23: trifluoromethane, R32: difluoromethane, R41: monofluoromethane), hydrochlorofluorocarbons (R22: chlorodifluoromethane, R21: dichloromonofluoromethane), and chlorofluorocarbons (R13: chlorotrifluoromethane, R11: trichloromonofluoromethane). Compared to hydrogen fluoride, the fluorinating agent tends to easily increase the pore volume of a fluorinated metal oxide catalyst, easily reduce catalyst degradation, and easily inhibit catalyst degradation even when the isomerization reaction is performed for a long time. Such fluorinating agents can be used singly or in a combination with two or more.

Although the fluorination conditions are not limited, the temperature is preferably -20°C to 400°C, and more preferably 0°C to 200°C from the viewpoint of easily reducing catalyst degradation and easily inhibiting catalyst degradation even when the isomerization reaction is performed for a long time.

For the same reason, the pressure in the fluorination conditions is preferably -50 kPa to 2,000 kPa, and more preferably 0 kPa to 1,000 kPa.

For the same reason, the time in the fluorination conditions is preferably 0.1 to 24 hours, and more preferably 1 to 12 hours.

### Examples of Catalyst Use

In the isomerization reaction step of the present disclosure, among catalysts, a fluorinated or non-fluorinated aluminum chloride catalyst, a fluorinated or non-fluorinated chromium oxide catalyst, a fluorinated or non-fluorinated alumina catalyst, or the like is preferably used from the viewpoint of conversion rate, selectivity, and yield.

When a fluorinated or non-fluorinated Lewis acid catalyst is used as a catalyst, the catalyst is preferably supported on a carrier. Examples of carriers that are preferably used include carbon, alumina (Al₂O₃), zirconia (ZrO₂), silica (SiO₂), and titania (TiO₂). Examples of carbons that can be used include activated carbon, amorphous carbon, graphite, and diamond.

In the isomerization reaction step, when the starting compound is subjected to an isomerization reaction in the presence of a catalyst, it is preferable to bring the catalyst in a solid state (solid phase) into contact with the starting compound. The catalyst can be in the form of powder or pellets, and a powder form is preferably used for a liquid phase reaction.

Preferably, the specific surface area (which may be referred to below as "BET specific surface area") of the catalyst measured by the BET method is typically 10 to 3000 m²/g, more preferably 10 to 2500 m²/g, even more preferably 20 to 2000 m²/g, and particularly preferably 30 to 1500 m²/g. A catalyst having a BET specific surface area within these numerical ranges does not have an overly low particle density, thus allowing the isomerization reaction to proceed efficiently to obtain the target compound at a high conversion rate and/or high yield, and/or with high selectivity.

### Reaction Temperature of Isomerization Reaction

In the isomerization reaction step of the present disclosure, the lower limit of the reaction temperature is preferably -20° or higher, more preferably 0°C or higher, and even more preferably 20°C or higher, from the viewpoint of allowing an isomerization reaction of the starting compound to proceed more efficiently to further increase the conversion rate and obtaining the target compound with higher selectivity.

In the isomerization reaction step, the upper limit of the reaction temperature is preferably 400°C or lower, more preferably 200°C or lower, and even more preferably 100°C or lower, from the viewpoint of allowing an isomerization reaction to proceed more efficiently to further increase the conversion rate and obtaining the target compound with higher selectivity, and from the viewpoint of suppressing a decrease in selectivity due to decomposition or polymerization of the reaction product.

In the isomerization reaction step, the reaction temperature is preferably room temperature (about 25°C).

In the isomerization reaction step, the reaction time can be appropriately set.

Even when a batch reaction is used, the contact time can be appropriately set.

The contact time means the time of contact between the starting compound (substrate) and the catalyst.

### Reaction Pressure of Isomerization Reaction

In the isomerization reaction step of the present disclosure, the reaction pressure for the isomerization reaction is preferably -0.05 MPa to 2 MPa, more preferably -0.01 MPa to 1 MPa, and even more preferably normal pressure to 0.5 MPa from the viewpoint of allowing the isomerization reaction to proceed more efficiently.

In the present disclosure, the pressure is gauge pressure unless indicated otherwise.

### Reaction Vessel of the Isomerization Reaction

In the isomerization reaction step of the present disclosure, the reactor into which the starting compound is placed with a catalyst and subjected to an isomerization reaction is not limited in its shape and structure as long as it can withstand the temperature and pressure described above. Preferable examples of reactors in which the isomerization reaction is performed include vertical reactors, horizontal reactors, multitubular reactors, and the like. The material of the reactor used for the isomerization reaction is preferably glass, stainless steel, iron, nickel, iron-nickel alloy, or the like.

### Liquid-phase Reaction

The isomerization reaction step of the present disclosure is preferably performed by a liquid-phase reaction, and can be carried out in a batch process or in a flow process in which the starting compound (substrate) is continuously fed into the reactor and the target compound is continuously withdrawn from the reactor.

Because the target compound remaining in the reactor suppresses the progression of the isomerization reaction, the isomerization reaction is preferably performed in a flow process.

### Liquid-phase Continuous Flow Process

The isomerization reaction step of the present disclosure is preferably performed in a liquid phase, and more preferably in a liquid-phase continuous flow process using a fixed-bed reactor. Performing the isomerization reaction in a liquid-phase continuous flow process can simplify the equipment, operation, etc., and is also economically advantageous.

In the isomerization step, the atmosphere in which the isomerization reaction is performed is preferably an inert gas atmosphere and/or a hydrogen fluoride atmosphere from the standpoint of suppressing catalyst degradation. Preferable examples of the inert gas include nitrogen, helium, and argon. Among the inert gases, nitrogen is preferably used from the viewpoint of cost reduction. The concentration of the inert gas is preferably 0 to 50 mol% of the gas component introduced into the reactor.

In the isomerization reaction step, the target compound can be obtained with higher selectivity by appropriately adjusting the reaction temperature and the reaction time (contact time) according to the catalyst.

### Purification Treatment

In the isomerization reaction step of the present disclosure, a purification treatment may be optionally performed in accordance with a common method after completion of the isomerization reaction to obtain the target compound.

### (3) Target Compound

The target compound of the present disclosure is 1,1,1,4,4,4-hexafluoro-2-butyne (CF₃C≡CCF₃).

In the isomerization reaction step, 1,1,2,3,4,4-hexafluoro-1,3-butadiene (CF₂=CF-CF=CF₂), which is the starting compound, is reacted to convert the two C-C double bonds of the starting compound into one C-C triple bond, thereby producing 1,1,1,4,4,4-hexafluoro-2-butyne (CF₃C≡CCF₃).

### [2] Composition Comprising Hexafluoro-2-butyne

(2-1) The composition of the present disclosure comprises:
(1) 1,1,1,4,4,4-hexafluoro-2-butyne (CF₃C≡CCF₃);
(2) 1,1,2,3,4,4-hexafluoro-1,3-butadiene (CF₂=CF-CF=CF₂); and
(3) (3-1) at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br).

In the method for producing 1,1,1,4,4,4-hexafluoro-2-butyne (CF₃C≡CCF₃) of the present disclosure, the product obtained by the isomerization reaction step may be in the form of a composition comprising 1,1,1,4,4,4-hexafluoro-2-butyne, which is the target compound in isomerized form, as well as 1,1,2,3,4,4-hexafluoro-1,3-butadiene (CF₂=CF-CF=CF₂), which is the starting compound, and at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br), which is used in the isomerization reaction step.

In the composition of the present disclosure, preferably, in terms of the composition described above, the content of the 1,1,1,4,4,4-hexafluoro-2-butyne (CF₃C=CCF₃)is 97 vol% or more and 99.99999 vol% or less, the content of the 1,1,2,3,4,4-hexafluoro-1,3-butadiene (CF₂=CF-CF=CF₂) is 0.01 vol% or more and 1 vol% or less, and the content of the olefin compound is 0.01 vol% or more and 1.5 vol% or less, based on the total amount of the composition.

The composition comprising hexafluoro-2-butyne of the present disclosure comprises an olefin compound, such as heptafluorobutene (C₄F₇H), and has improved storage stability. Thus, even under harsh maritime transportation conditions in containers or the like (e.g., temperature rise during transportation), polymerization (formation of polymers) or decomposition (formation of decomposed products) of hexafluoro-2-butyne is suppressed, or formation of acid components is suppressed, whereby the composition comprising hexafluoro-2-butyne can be stably stored and transported.

### Content of 1,1,1,4,4,4-Hexafluoro-2-butyne

In the composition of the present disclosure, the content of 1,1,1,4,4,4-hexafluoro-2-butyne (CF₃C=CCF₃)is preferably 95 vol% or more and 99.99999 vol% or less, more preferably 96 vol% or more and 99.9999 vol% or less, even more preferably 97 vol% or more and 99.9995 vol% or less, and particularly preferably 99.8 vol% or more and 99.999 vol% or less, based on the total amount of the composition.

### Content of 1,1,2,3,4,4-Hexafluoro-1,3-butadiene

In the composition of the present disclosure, the content of 1,1,2,3,4,4-hexafluoro-1,3-butadiene (CF₂=CF-CF=CF₂) is preferably 0.01 vol% or more and 1 vol% or less, more preferably 0.05 vol% or more and 0.95 vol% or less, even more preferably 0.1 vol% or more and 0.90 vol% or less, and particularly preferably 0.15 vol% or more and 0.85 vol% or less, based on the total amount of the composition.

### Content of Olefin Compound

In the composition of the present disclosure, the content of the olefin compound (when used singly or the total content when the olefin compound is used in a combination of two or more) is preferably 0.01 vol% or more and 1.5 vol% or less, more preferably 0.03 vol% or more and 1.0 vol% or less, even more preferably 0.05 vol% or more and 0.5 vol% or less, and particularly preferably 0.07 vol% or more and 0.20 vol% or less, based on the total amount of the composition.

(2-2) The composition of the present disclosure comprises:
(1) 1,1,1,4,4,4-hexafluoro-2-butyne (CF₃C≡CCF₃);
(2) 1,1,2,3,4,4-hexafluoro-1,3-butadiene (CF₂=CF-CF=CF₂); and
(3) (3-2) oxygen.

In the method for producing 1,1,1,4,4,4-hexafluoro-2-butyne (CF₃C≡CCF₃) of the present disclosure, the product obtained by the isomerization reaction step may be in the form of a composition comprising 1,1,1,4,4,4-hexafluoro-2-butyne, which is the target compound in isomerized form, as well as 1,1,2,3,4,4-hexafluoro-1,3-butadiene (CF₂=CF-CF=CF₂), which is the starting compound, and oxygen (O₂), which is used in the isomerization reaction step.

In the composition of the present disclosure, preferably, in terms of the composition described above, the content of the 1,1,1,4,4,4-hexafluoro-2-butyne (CF₃C≡CCF₃) is 95 vol% or more and 99.99999 vol% or less, the content of the 1,1,2,3,4,4-hexafluoro-1,3-butadiene (CF₂=CF-CF=CF₂) is 0.01 vol% or more and 1 vol% or less, and the content of the oxygen is 0.01 vol ppm or more and 10 vol ppm or less, based on the total amount of the composition.

The composition comprising hexafluoro-2-butyne of the present disclosure comprises oxygen and has improved storage stability. Thus, even under harsh maritime transportation conditions in containers or the like (e.g., temperature rise during transportation), polymerization (formation of polymers) or decomposition (formation of decomposed products) of hexafluoro-2-butyne is suppressed, or formation of acid components is suppressed, whereby the composition comprising hexafluoro-2-butyne can be stably stored and transported.

### Content of 1,1,1,4,4,4-Hexafluoro-2-butyne

In the composition of the present disclosure, the content of 1,1,1,4,4,4-hexafluoro-2-butyne (CF₃C=CCF₃)is preferably 95 vol% or more and 99.99999 vol% or less, more preferably 96 vol% or more and 99.9999 vol% or less, even more preferably 97 vol% or more and 99.9995 vol% or less, and particularly preferably 98 vol% or more and 99.999 vol% or less, based on the total amount of the composition.

### Content of 1,1,2,3,4,4-Hexafluoro-1,3-butadiene

In the composition of the present disclosure, the content of 1,1,2,3,4,4-hexafluoro-1,3-butadiene (CF₂=CF-CF=CF₂) is preferably 0.01 vol% or more and 1 vol% or less, more preferably 0.05 vol% or more and 0.95 vol% or less, even more preferably 0.1 vol% or more and 0.90 vol% or less, and particularly preferably 0.15 vol% or more and 0.85 vol% or less, based on the total amount of the composition.

### Content of Oxygen

In the composition of the present disclosure, the content of oxygen is preferably 0.01 vol ppm or more and 10 vol ppm or less, more preferably 0.1 vol ppm or more and 9.5 vol ppm or less, even more preferably 0.5 vol ppm or more and 7.5 vol ppm or less, and particularly preferably 1 vol ppm or more and 5 vol ppm or less, based on the total amount of the composition.

(2-3) The composition of the present disclosure comprises:
(1) 1,1,1,4,4,4-hexafluoro-2-butyne (CF₃C≡CCF₃);
(2) 1,1,2,3,4,4-hexafluoro-1,3-butadiene (CF₂=CF-CF=CF₂); and
(3) (3-3) water.

In the method for producing 1,1,1,4,4,4-hexafluoro-2-butyne (CF₃C≡CCF₃) of the present disclosure, the product obtained by the isomerization reaction step may be in the form of a composition comprising 1,1,1,4,4,4-hexafluoro-2-butyne, which is the target compound in isomerized form, as well as 1,1,2,3,4,4-hexafluoro-1,3-butadiene (CF₂=CF-CF=CF₂), which is the starting compound, and water, which is used in the isomerization reaction step.

In the composition of the present disclosure, preferably, in terms of the composition described above, the content of the 1,1,1,4,4,4-hexafluoro-2-butyne (CF₃C≡CCF₃) is 95 vol% or more and 99.99999 vol% or less, the content of the 1,1,2,3,4,4-hexafluoro-1,3-butadiene (CF₂=CF-CF=CF₂) is 0.01 vol% or more and 1 vol% or less, and the content of the water is 0.01 vol ppm or more and 30 vol ppm or less, based on the total amount of the composition.

The composition comprising hexafluoro-2-butyne of the present disclosure comprises water and has improved storage stability. Thus, even under harsh maritime transportation conditions in containers or the like (e.g., temperature rise during transportation), polymerization (formation of polymers) or decomposition (formation of decomposed products) of hexafluoro-2-butyne is suppressed, or formation of acid components is suppressed, whereby the composition comprising hexafluoro-2-butyne can be stably stored and transported.

### Content of 1,1,1,4,4,4-Hexafluoro-2-butyne

In the composition of the present disclosure, the content of 1,1,1,4,4,4-hexafluoro-2-butyne (CF₃C=CCF₃)is preferably 95 vol% or more and 99.99999 vol% or less, more preferably 96 vol% or more and 99.9999 vol% or less, even more preferably 97 vol% or more and 99.9995 vol% or less, and particularly preferably 98 vol% or more and 99.999 vol% or less, based on the total amount of the composition.

### Content of 1,1,2,3,4,4-Hexafluoro-1,3-butadiene

In the composition of the present disclosure, the content of 1,1,2,3,4,4-hexafluoro-1,3-butadiene (CF₂=CF-CF=CF₂) is preferably 0.01 vol% or more and 1 vol% or less, more preferably 0.05 vol% or more and 0.95 vol% or less, even more preferably 0.1 vol% or more and 0.90 vol% or less, and particularly preferably 0.15 vol% or more and 0.85 vol% or less, based on the total amount of the composition.

### Content of Water

In the composition of the present disclosure, the water content is preferably 0.01 vol ppm or more and 30 vol ppm or less, more preferably 0.1 vol ppm or more and 25 vol ppm or less, even more preferably 0.5 vol ppm or more and 20 vol ppm or less, and particularly preferably 1 vol ppm or more and 10 vol ppm or less, based on the total amount of the composition.

### [3] Use of Composition Comprising Hexafluoro-2-butyne

The composition of the present disclosure is preferably used as an etching gas, a refrigerant, a heat-transfer medium, a deposit gas, a building block for organic synthesis, or a cleaning gas.

The composition is preferably used, for example, as an etching gas for forming state-of-the-art microstructures, such as semiconductors and liquid crystals, as well as a refrigerant, a heat-transfer medium, and the like. Preferably, the composition is effectively used for various applications, such as a deposit gas, a building block for organic synthesis, and a cleaning gas.

The deposit gas means a gas for depositing an etching-resistant polymer layer.

The "building block for organic synthesis" means a substance that can serve as a precursor of a compound that has a highly reactive skeleton. When a composition comprising 1,1,1,4,4,4-hexafluoro-2-butyne (CF₃C≡CCF₃) is reacted with a fluorine-containing organosilicon compound, such as CF₃Si(CH₃)₃, a fluoroalkyl group, such as a CF₃ group, is introduced to convert the composition into a substance that can be used as a cleaning agent or a fluorine-containing pharmaceutical intermediate.

### Examples

The present invention is described in detail below with reference to Examples and Comparative Examples. The present invention is not limited to these Examples.

### Example 1: In the Presence of Olefin Compound

### [1] Production of Hexafluoro-2-butyne

Starting compound: 1,1,2,3,4,4-hexafluoro-1,3-butadiene (CF₂=CF-CF=CF₂)
Target compound: 1,1,1,4,4,4-hexafluoro-2-butyne (CF₃C=CCF₃) Olefin compound:
   Octafluorobutene (C₄F₈): octafluoro-1-butene, (E/Z)-octafluoro-2-butene
   Heptafluorobutene (C₄F₇H): 1,1,2,3,3,4,4-heptafluoro-1-butene, (E/Z)-1,1,1,2,4,4,4-heptafluoro-2-butene
   Pentafluorobutadiene (C₄F₅H): 2H-pentafluoro-1,3-butadiene
   Chloropentafluorobutadiene (C₄F₅Cl): (E/Z)-1-chloropentafluoro-1,3-butadiene
   Bromotrifluoroethylene (C₂F₃Br): 1-bromo-1,2,2-trifluoroethene

Gas chromatography: produced by Shimadzu Corporation, trade name: GC-2014
NMR: produced by JEOL Ltd., trade name: 400YH

### (1) Liquid-phase Isomerization Reaction

In a reaction by liquid-phase flow process, the pressure was set to normal pressure, and 1,1,2,3,4,4-hexafluoro-1,3-butadiene (starting compound) was introduced into a reactor in the presence of an olefin compound such that the molar ratio of the Lewis acid catalyst to the staring compound (Lewis acid catalyst/starting compound) was 0.01, 0.1, or 0.3.

The reaction was allowed to proceed in a liquid-phase reaction.

The catalyst was added to a SUS vessel serving as the reactor, and drying was performed under a nitrogen atmosphere. The pressure was then reduced to vacuum, and after the temperature reached 25°C, R22 (chlorodifluoromethane) as a fluorinating agent was introduced. Subsequently, the temperature was lowered to 25°C (room temperature), and a fluorination reaction was initiated. Thereafter, 1,1,2,3,4,4-hexafluoro-1,3-butadiene, i.e., the starting compound, was introduced such that the molar ratio was 0.1.

The reactor was set to 25°C (room temperature), and an isomerization reaction was initiated.

After the start of the reaction, the gas was collected.

After completion of the reaction, mass spectrometry was performed based on gas chromatography-mass spectrometry (GC/MS) by using gas chromatography, and structural analysis was performed based on an NMR spectrum by NMR. The mass spectrometry and the structural analysis confirmed that 1,1,1,4,4,4-hexafluoro-2-butyne was obtained as the target compound.

### ND: Not Detected

**Table 1**

| Table 1 | Initial composition | Unit: | vol% | | | | | |
|---|---|---|---|---|---|---|---|---|
| | Starting compound | Olefin compound | | | | | | |
| | C₄F₆ | C₄F₈ | C₄F₅H | C₄F₅Cl | C₄F₇H | C₂F₃Br | Others | HF |
| Ex. 1-1 | 99.90 | 0.001 | 0.01 | 0.01 | 0.01 | 0.01 | 0.059 | ND |
| Ex. 1-2 | 99.99 | ND | ND | ND | 0.01 | ND | 0.00 | ND |
| Ex. 1-3 | 99.99 | ND | ND | ND | ND | 0.01 | 0.00 | ND |
| Ex. 1-4 | 99.97 | 0.01 | 0.01 | 0.01 | 0.001 | 0.001 | 0.00 | ND |
| Ex. 1-5 | 99.94 | 0.01 | ND | 0.04 | ND | ND | 0.01 | ND |
| Ex. 1-6 | 99.1 | 0.12 | 0.04 | 0.17 | 0.20 | 0.03 | 0.34 | ND |
| Ref. Ex. 1-1 | 99.9993 | ND | ND | ND | 0.0007 | ND | ND | ND |
| Ref. Ex. 1-2 | 98.2 | 0.48 | 0.07 | 0.03 | 0.19 | 0.45 | 0.58 | ND |

**Table 2**

| Table 2 | Immediately after completion of the reaction | | Unit**:** vol% | | | | |
|---|---|---|---|---|---|---|---|
| | Target compound | Starting compound | Olefin compound | | | | |
| | CF₃C≡CCF₃ | C₄F₆ | C₄F₅Cl | C₄F₇H | C₂F₃Br | Others | HF |
| Ex. 2-1 | 99.4 | 0.38 | 0.16 | 0.02 | 0.03 | 0.01 | ND |
| Ex. 2-2 | 99.4 | 0.38 | 0.15 | 0.02 | ND | 0.05 | ND |
| Ex. 2-3 | 99.5 | 0.30 | 0.13 | ND | 0.02 | 0.05 | ND |
| Ex. 2-4 | 99.4 | 0.50 | 0.08 | ND | ND | 0.02 | ND |
| Ex. 2-5 | 98.65 | 0.92 | 0.088 | 0.112 | ND | 0.229 | ND |
| Ex. 2-6 | 97.5 | 0.97 | 0.29 | 0.72 | 0.22 | 0.296 | ND |
| Ref. Ex. 2-1 | 99.991 | 0.0083 | ND | 0.0007 | ND | ND | ND |
| Ref. Ex. 2-2 | 96.3 | 2.10 | 0.17 | 0.16 | 0.05 | 0.22 | ND |

**Table 3**

| Table 3 | After one week of storage in a storage tank | | Unit: vol% | | | | | |
|---|---|---|---|---|---|---|---|---|
| | Target compound | Starting compound | Olefin compound | | | | | Pipe cross-section status Presence of pipe clogaing |
| | CF₃C≡CCF₃ | C₄F₆ | C₄F₅Cl | C₄F₇H | C₂F₃Br | Others | HF | |
| Ex. 3-1 | 99.5 | 0.1 | 0.11 | ND | ND | 0.29 | ND | No |
| Ex. 3-2 | 99.5 | 0.14 | 0.11 | ND | ND | 0.25 | ND | No |
| Ex. 3-3 | 99.5 | 0.22 | 0.11 | ND | ND | 0.17 | ND | No |
| Ex. 3-4 | 99.4 | 0.5 | 0.08 | ND | ND | 0.02 | ND | No |
| Ex. 3-5 | 98.65 | 0.82 | 0.088 | 0.012 | ND | 0.429 | ND | No |
| Ex. 3-6 | 97.5 | 0.33 | 0.63 | 0.99 | ND | 0.55 | ND | No |
| Ref. Ex. 3-1 | 99.987 | 0.0055 | ND | 0.0005 | ND | 0.0070 | >100 | Yes |
| Ref. Ex. 3-2 | 98.1 | 0.55 | 0.21 | 0.16 | 0.16 | 0.82 | >100 | Yes |

| | | | | | | | | |
|---|---|---|---|---|---|---|---|---|
| ND: Not Detected | | | | | | | | |

### [2] Storage Stability of Composition Comprising Hexafluoro-2-butyne

In the production method of the Examples, the concentrations of the olefin compounds included in the starting material were adjusted, whereby polymerization (e.g., dimerization) of 1,1,1,4,4,4-hexafluoro-2-butyne (CF₃C≡CCF₃), i.e., the target compound, was suppressed, or the formation of acid components was suppressed.

In the production method of the Examples, the concentrations of the olefin compounds in the crude products of 1,1,1,4,4,4-hexafluoro-2-butyne obtained after the reaction were adjusted, and the crude products of 1,1,1,4,4,4-hexafluoro-2-butyne after the reaction had improved storage stability during storage in a storage tank.

### Example 2: In the presence of Oxygen

### [1] Production of Hexafluoro-2-butyne

Starting compound: 1,1,2,3,4,4-hexafluoro-1,3-butadiene (CF₂=CF-CF=CF₂)
Target compound: 1,1,1,4,4,4-hexafluoro-2-butyne (CF₃C=CCF₃) Oxygen

The starting compound and the target compound were analyzed using gas chromatography (produced by Shimadzu Corporation, trade name: GC-2014, detector: FID) and NMR (produced by JEOL Ltd., trade name: 400YH).

Oxygen was analyzed using gas chromatography (produced by Shimadzu Corporation, trade name: GC-2030, detector: BID).

### (1) Liquid-phase Batch Isomerization Reaction

In a liquid-phase reaction, the pressure was set to normal pressure, and 1,1,2,3,4,4-hexafluoro-1,3-butadiene (starting compound) was introduced into a reactor in the presence of oxygen such that the molar ratio of the Lewis acid catalyst to the staring compound (Lewis acid catalyst/starting compound) was 0.01, 0.1, or 0.3.

The reaction was allowed to proceed in a liquid-phase batch reaction.

The catalyst was added to a SUS vessel serving as the reactor, and drying was performed under a nitrogen atmosphere. The pressure was then reduced to vacuum, and after the temperature reached 25°C, R22 (chlorodifluoromethane) as a fluorinating agent was introduced. Subsequently, the temperature was lowered to 25°C (room temperature), and a fluorination reaction was initiated. Thereafter, 1,1,2,3,4,4-hexafluoro-1,3-butadiene, i.e., the starting compound, was introduced such that the molar ratio was 0.1.

The reactor was set to 25°C (room temperature) or heated at 100°C, and an isomerization reaction was initiated.

After completion of the reaction, the gas was collected according to a common method.

After completion of the reaction, mass spectrometry was performed based on gas chromatography-mass spectrometry (GC/MS) by using gas chromatography, and structural analysis was performed based on an NMR spectrum by NMR. The mass spectrometry and the structural analysis confirmed that 1,1,1,4,4,4-hexafluoro-2-butyne was obtained as the target compound.

**Table 4**

| Table 4 | Composition of the starting compound used in the reaction | | | |
|---|---|---|---|---|
| | Initial composition of the starting material: compound | | | |
| | Oxygen | Water | C₄F₆ | Others |
| | vol ppm | vol ppm | vol% | vol% |
| Ref. Ex. 4-1 | 31 | 32 | 99.911 | 0.089 |
| Ref. Ex. 4-2 | 18 | 16 | 94.165 | 5.835 |
| Ex. 4-3 | 9 | 9 | 99.995 | 0.005 |
| Ex. 4-4 | 1 | 0.05 | 96.585 | 3.415 |
| Ex. 4-5 | 0.1 | 1.3 | 99.990 | 0.010 |
| Ex. 4-6 | 2.2 | 43 | 99.730 | 0.270 |
| Ex. 4-7 | 9.8 | 0.02 | 95.678 | 4.322 |
| Ex. 4-8 | 0.6 | 11 | 96.633 | 3.367 |
| Ref. Ex. 4-3 | 13 | 70 | 94.525 | 5.475 |
| Ref. Ex. 4-4 | 0.005 | 0.005 | 94.500 | 5.500 |

**Table 5**

| Table 5 | Composition after completion of the reaction | | | | | |
|---|---|---|---|---|---|---|
| | Immediately after completion of the reaction | | | | | |
| | Target compound | Starting compound | | | | |
| | CF₃C≡CCF₃ | C₄F₆ | Others | Oxygen | Water | HF |
| | vol% | vol% | vol% | vol ppm | vol ppm | vol ppm |
| Ref. Ex. 5-1 | 99.511 | 0.4 | 0.089 | 29 | 28 | ND |
| Ref. Ex. 5-2 | 93.185 | 0.98 | 5.835 | 17 | 9 | ND |
| Ex. 5-3 | 99.835 | 0.16 | 0.005 | 9.2 | 5 | ND |
| Ex. 5-4 | 96.255 | 0.33 | 3.415 | 1.2 | 0.04 | ND |
| Ex. 5-5 | 99.89 | 0.1 | 0.01 | 0.1 | 0.9 | ND |
| Ex. 5-6 | 99.43 | 0.3 | 0.27 | 3.3 | 21 | ND |
| Ex. 5-7 | 95.698 | 0.98 | 3.322 | 9.7 | 0.02 | ND |
| Ex. 5-8 | 96.623 | 0.01 | 3.367 | 0.7 | 7 | ND |
| Ref. Ex. 5-3 | 92.725 | 1.8 | 5.475 | 12 | 32 | ND |
| Ref. Ex. 5-4 | 93.177 | 1.6 | 5.223 | 0.004 | 0.002 | ND |

**Table 6**

| Table 6 | Composition one week after completion of the reaction | | | | | |
|---|---|---|---|---|---|---|
| | One week after completion of the reaction | | | | | |
| | Target compound | Starting compound | | | | Pipe cross-section status Presence of pipe clogging |
| | CF₃C≡CCF₃ | C₄F₆ | Others | Decomposed product | HF | |
| | vol% | vol% | vol% | vol ppm | vol ppm | |
| Ref. Ex. 6-1 | 99.511 | 0.31 | 0.009 | 6 | >100 | Yes |
| Ref. Ex. 6-2 | 93.185 | 0.91 | 5.887 | 6 | ND | No |
| Ex. 6-3 | 99.835 | 0.16 | 0.005 | 0 | ND | No |
| Ex. 6-4 | 96.255 | 0.33 | 3.415 | 0 | ND | No |
| Ex. 6-5 | 99.900 | 0.09 | 0.01 | 10 | ND | No |
| Ex. 6-6 | 99.430 | 0.3 | 0.27 | 0 | ND | No |
| Ex. 6-7 | 94.698 | 0.98 | 4.322 | 0.6 | ND | No |
| Ex. 6-8 | 96.623 | 0.01 | 3.367 | 0.4 | ND | No |
| Ref. Ex. 6-3 | 93.802 | 1.2 | 4.998 | 10 | >100 | Yes |
| Ref. Ex. 6-4 | 94.602 | 0.9 | 4.498 | 13 | >100 | Yes |

### [2] Storage Stability of Composition Comprising Hexafluoro-2-butyne

In the production method of the Examples, the concentration of oxygen included in the starting material was adjusted, whereby polymerization (e.g., dimerization) of 1,1,1,4,4,4-hexafluoro-2-butyne (CF₃C≡CCF₃), i.e., the target compound, was suppressed, or the formation of acid components was suppressed.

In the production method of the Examples, the concentration of oxygen in the crude products of 1,1,1,4,4,4-hexafluoro-2-butyne obtained after the reaction was adjusted, and the crude products of 1,1,1,4,4,4-hexafluoro-2-butyne after the reaction had improved storage stability during storage in a storage tank.

### Example 3: In the Presence of Water

### [1] Production of Hexafluoro-2-butyne

Starting compound: 1,1,2,3,4,4-hexafluoro-1,3-butadiene (CF₂=CF-CF=CF₂)
Target compound: 1,1,1,4,4,4-hexafluoro-2-butyne (CF₃C=CCF₃) Water

The starting compound and the target compound were analyzed using gas chromatography (produced by Shimadzu Corporation, trade name: GC-2014, detector: FID) and NMR (produced by JEOL Ltd., trade name: 400YH).

Water was analyzed using a moisture analyzer (Quartz crystal microbalance (QCM) process moisture analyzer, QMA, produced by Michell Instruments Japan) or CRDS (produced by Tiger Optics).

### (1) Liquid-phase Batch Isomerization Reaction

In a reaction by liquid-phase flow process, the pressure was set to normal pressure, and 1,1,2,3,4,4-hexafluoro-1,3-butadiene (starting compound) was introduced into a reactor in the presence of water such that the molar ratio of the Lewis acid catalyst to the staring compound (Lewis acid catalyst/starting compound) was 0.01, 0.1, or 0.3.

The reaction was allowed to proceed in a liquid-phase batch mode.

The catalyst was added to a SUS vessel serving as the reactor, and drying was performed under a nitrogen atmosphere. The pressure was then reduced to vacuum, and after the temperature reached 25°C, R22 (chlorodifluoromethane) as a fluorinating agent was introduced. Subsequently, the temperature was lowered to 25°C (room temperature), and a fluorination reaction was initiated. Thereafter, 1,1,2,3,4,4-hexafluoro-1,3-butadiene, i.e., the starting compound, was introduced such that the molar ratio was 0.1.

The reactor was set to 25°C (room temperature) or heated at 100°C, and an isomerization reaction was initiated.

After completion of the reaction, the gas was collected according to a common method.

After completion of the reaction, mass spectrometry was performed based on gas chromatography-mass spectrometry (GC/MS) by using gas chromatography, and structural analysis was performed based on an NMR spectrum by NMR. The mass spectrometry and the structural analysis confirmed that 1,1,1,4,4,4-hexafluoro-2-butyne was obtained as the target compound.

**Table 7**

| Table 7 | Composition of the starting compound used in the reaction | | | |
|---|---|---|---|---|
| | Initial composition: Starting compound | | | |
| | Oxygen | Water | C₄F₆ | Others |
| | vol ppm | vol ppm | vol% | vol% |
| Ref. Ex. 7-1 | 31 | 32 | 99.911 | 0.089 |
| Ref. Ex. 7-2 | 18 | 16 | 94.165 | 5.835 |
| Ex. 7-3 | 9 | 9 | 99.995 | 0.005 |
| Ex. 7-4 | 1 | 0.05 | 96.585 | 3.415 |
| Ex. 7-5 | 0.1 | 1.3 | 99.990 | 0.010 |
| Ex. 7-6 | 2.2 | 43 | 99.730 | 0.270 |
| Ex. 7-7 | 9.8 | 0.02 | 95.678 | 4.322 |
| Ex. 7-8 | 0.6 | 11 | 96.633 | 3.367 |
| Ref. Ex. 7-3 | 13 | 70 | 94.525 | 5.475 |
| Ref. Ex. 7-4 | 0.005 | 0.005 | 94.500 | 5.500 |

**Table 8**

| Table 8 | Composition after completion of the reaction | | | | | |
|---|---|---|---|---|---|---|
| | Immediately after completion of the reaction | | | | | |
| | Target compound | Starting compound | | | | |
| | CF₃C≡CCF₃ | C₄F₆ | Others | Oxygen | Water | HF |
| | vol% | vol% | vol% | vol ppm | vol ppm | vol ppm |
| Ref. Ex. 8-1 | 99.511 | 0.4 | 0.089 | 29 | 28 | ND |
| Ref. Ex. 8-2 | 93.185 | 0.98 | 5.835 | 17 | 9 | ND |
| Ex. 8-3 | 99.835 | 0.16 | 0.005 | 9.2 | 5 | ND |
| Ex. 8-4 | 96.255 | 0.33 | 3.415 | 1.2 | 0.04 | ND |
| Ex. 8-5 | 99.89 | 0.1 | 0.01 | 0.1 | 0.9 | ND |
| Ex. 8-6 | 99.43 | 0.3 | 0.27 | 3.3 | 21 | ND |
| Ex. 8-7 | 95.698 | 0.98 | 3.322 | 9.7 | 0.02 | ND |
| Ex. 8-8 | 96.623 | 0.01 | 3.367 | 0.7 | 7 | ND |
| Ref. Ex. 8-3 | 92.725 | 1.8 | 5.475 | 12 | 32 | ND |
| Ref. Ex. 8-4 | 93.177 | 1.6 | 5.223 | 0.004 | 0.002 | ND |

**Table 9**

| Table 9 | Composition one week after completion of the reaction | | | | | |
|---|---|---|---|---|---|---|
| | One week after completion of the reaction | | | | | |
| | Target compound | Starting compound | | | | Pipe cross-section status Presence of pipe dogging |
| | CF₃C≡CCF₃ | C₄F₆ | Others | Decomposed product | HF | |
| | vol% | vol% | vol% | vol ppm | vol ppm | |
| Ref. Ex. 9-1 | 99.511 | 0.31 | 0.009 | 6 | >100 | Yes |
| Ref. Ex. 9-2 | 93.185 | 0.91 | 5.887 | 6 | ND | No |
| Ex. 9-3 | 99.835 | 0.16 | 0.005 | 0 | ND | No |
| Ex. 9-4 | 96.255 | 0.33 | 3.415 | 0 | ND | No |
| Ex. 9-5 | 99.900 | 0.09 | 0.01 | 10 | ND | No |
| Ex. 9-6 | 99.430 | 0.3 | 0.27 | 0 | ND | No |
| Ex. 9-7 | 94.698 | 0.98 | 4.322 | 0.6 | ND | No |
| Ex. 9-8 | 96.623 | 0.01 | 3.367 | 0.4 | ND | No |
| Ref. Ex. 9-3 | 93.802 | 1.2 | 4.998 | 10 | >100 | Yes |
| Ref. Ex. 9-4 | 94.602 | 0.9 | 4.498 | 13 | >100 | Yes |

### [2] Storage Stability of Composition Comprising Hexafluoro-2-butyne

In the production method of the Examples, the concentration of water included in the starting material was adjusted, whereby polymerization (e.g., dimerization) of 1,1,1,4,4,4-hexafluoro-2-butyne (CF₃C≡CCF₃), i.e., the target compound, was suppressed, or the formation of acid components was suppressed.

In the production method of the Examples, the concentration of water in the crude products of 1,1,1,4,4,4-hexafluoro-2-butyne obtained after the reaction was adjusted, and the crude products of 1,1,1,4,4,4-hexafluoro-2-butyne after the reaction had improved storage stability during storage in a storage tank.

### [3] Industrial Applicability

In the method for producing 1,1,1,4,4,4-hexafluoro-2-butyne (CF₃C≡CCF₃) of the present disclosure, the concentration of at least one component selected from the group consisting of an olefin compound, oxygen (O₂), and water included in the starting material is adjusted, whereby polymerization (e.g., dimerization) of the halogenated alkyne compound or formation of acid components can be suppressed, thereby improving the storage stability of the crude product after the reaction.

In the method for producing 1,1,1,4,4,4-hexafluoro-2-butyne (CF₃C≡CCF₃) of the present disclosure, the concentration of at least one component selected from the group consisting of an olefin compound, oxygen (O₂), and water in the crude 1,1,1,4,4,4-hexafluoro-2-butyne obtained after the reaction is adjusted; whereby decomposition reactions, as well as formation of polymers and acid components, can be suppressed during storage in a storage tank, thereby suppressing, for example, clogging of pipes and corrosion of equipment during storage in the storage tank.

In the production of hexafluoro-2-butyne of the present disclosure, hexafluoro-1,3-butadiene (CF₂=CF-CF=CF₂) is subjected to an isomerization reaction in the presence of an olefin compound, such as heptafluorobutene (C₄F₇H), in the presence of oxygen, or in the presence of water, whereby polymerization (formation of polymers) or decomposition (formation of decomposed products) of hexafluoro-2-butyne is suppressed, or formation of acid components is suppressed during storage or transportation of the produced composition comprising hexafluoro-2-butyne in cylinders or the like, thereby a decrease in purity of hexafluoro-2-butyne is suppressed while maintaining the storage stability of the composition comprising hexafluoro-2-butyne.

The composition comprising hexafluoro-2-butyne of the present disclosure comprises at least one component selected from the group consisting of an olefin compound, such as heptafluorobutene (C₄F₇H), oxygen (O₂), and water, and has improved storage stability. Thus, even under harsh maritime transportation conditions in containers or the like (e.g., temperature rise during transportation), polymerization (formation of polymers) or decomposition (formation of decomposed products) of hexafluoro-2-butyne is suppressed, or formation of acid components is suppressed, whereby the composition comprising hexafluoro-2-butyne can be stably stored and transported.

## Claims

1. A method for producing (1) 1,1,1,4,4,4-hexafluoro-2-butyne (CF₃C≡CCF₃), comprising
subjecting (2) 1,1,2,3,4,4-hexafluoro-1,3-butadiene (CF₂=CF-CF=CF₂) to an isomerization reaction
(3) in the presence of
(3-1) at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br),
(3-2) oxygen, or
(3-2) water.

2. The production method according to claim 1, wherein in the isomerization reaction step,
when the isomerization reaction step is performed (3) in the presence of (3-1) the olefin compound, the content of the 1,1,2,3,4,4-hexafluoro-1,3-butadiene (CF₂=CF-CF=CF₂) is 99.0 vol% or more and 99.999 vol% or less, and the content of the olefin compound is 0.001 vol% or more and 1 vol% or less, based on the total amount of a composition comprising the 1,1,2,3,4,4-hexafluoro-1,3-butadiene (CF₂=CF-CF=CF₂) and the olefin compound;
when the isomerization reaction step is performed (3) in the presence of (3-2) the oxygen, the content of the 1,1,2,3,4,4-hexafluoro-1,3-butadiene (CF₂=CF-CF=CF₂) is 95 vol% or more and 99.999 vol% or less, and the content of the oxygen is 0.01 vol ppm or more and 10 vol ppm or less, based on the total amount of a composition comprising the 1,1,2,3,4,4-hexafluoro-1,3-butadiene (CF₂=CF-CF=CF₂) and the oxygen; or
when the isomerization reaction step is performed (3) in the presence of (3-3) the water, the content of the 1,1,2,3,4,4-hexafluoro-1,3-butadiene (CF₂=CF-CF=CF₂) is 95 vol% or more and 99.999 vol% or less, and the content of the water is 0.01 vol ppm or more and 50 vol ppm or less, based on the total amount of a composition comprising the 1,1,2,3,4,4-hexafluoro-1,3-butadiene (CF₂=CF-CF=CF₂) and the water.

3. The production method according to claim 1, wherein the isomerization reaction step is performed in a liquid phase.

4. A composition comprising:
(1) 1,1,1,4,4,4-hexafluoro-2-butyne (CF₃C≡CCF₃);
(2) 1,1,2,3,4,4-hexafluoro-1,3-butadiene (CF₂=CF-CF=CF₂); and
(3) at least one component selected from the group consisting of
(3-1) at least one olefin compound selected from the group consisting of octafluorobutene (C₄F₈), heptafluorobutene (C₄F₇H), pentafluorobutadiene (C₄F₅H), chloropentafluorobutadiene (C₄F₅Cl), and bromotrifluoroethylene (C₂F₃Br),
(3-2) oxygen, and
(3-3) water.

5. The composition according to claim 4,
wherein based on the total amount of the composition,
the content of (1) the 1,1,1,4,4,4-hexafluoro-2-butyne (CF₃C≡CCF₃) is 95 vol% or more and 99.99999 vol% or less,
the content of (2) the 1,1,2,3,4,4-hexafluoro-1,3-butadiene (CF₂=CF-CF=CF₂) is 0.01 vol% or more and 1 vol% or less, and
as (3) the at least one component,
when the composition comprises (3-1) the olefin compound, the content of the olefin compound is 0.01 vol% or more and 1.5 vol% or less,
when the composition comprises (3-2) the oxygen, the content of the oxygen is 0.01 vol ppm or more and 10 vol ppm or less; or
when the composition comprises (3-3) the water, the content of the water is 0.01 vol ppm or more and 30 vol ppm or less.

6. The composition according to claim 4 for use as an etching gas, a refrigerant, a heat-transfer medium, a deposit gas, a building block for organic synthesis, or a cleaning gas.
